(19) **Europäisches Patentamt**
**European Patent Office**
**Office européen des brevets**

(11) **EP 0 808 640 B1**

(12) **EUROPEAN PATENT SPECIFICATION**

(45) Date of publication and mention
of the grant of the patent:
**02.05.2003 Bulletin 2003/18**

(51) Int Cl.$^7$: **A61N 5/06**

(21) Application number: **96307299.6**

(22) Date of filing: **07.10.1996**

(54) **Far-infrared radiator and method of radiating far-infrared rays**

Strahlungsquelle im Fern-Infrarot-Bereich und Verfahren zum Bestrahlen im Fern-Infrarot-Bereich

Emetteur de radiations dans l'infra-rouge lointain et méthode pour émettre des radiations dans l'infra-rouge lointain

(84) Designated Contracting States:
**BE DE GB**

(30) Priority: **24.05.1996 JP 12988896**

(43) Date of publication of application:
**26.11.1997 Bulletin 1997/48**

(73) Proprietor: **Kabushiki Kaisha Dairin Shoji**
**Sakai-shi, Osaka-fu 591 (JP)**

(72) Inventors:
• **Nomura, Harehiko**
**Tsukuba-shi, Ibaraki-ken 305 (JP)**

• **Hayashi, Yukiko**
**Sakai-shi, Osaka-fu 591 (JP)**

(74) Representative: **Brewster, Andrea Ruth et al**
**MEWBURN ELLIS**
**York House**
**23 Kingsway**
**London WC2B 6HP (GB)**

(56) References cited:
**WO-A-95/19809**

EP 0 808 640 B1

## Description

The present invention relates to a far-infrared radiator and a method of radiating far-infrared rays for radiating far-infrared rays in a particular wavelength range.

Various kinds of heaters have conventionally been developed for heating. US-A-4 591 701 discloses a heat radiator including a ceramic sheet covered by a carbon particle layer. Especially, the sheet-like heat generating elements which use the carbon fiber (hereinafter, referred to as a carbon fiber sheet-like heat generating element) have attracted special interest recently as heat generators for radiating the far-infrared rays. The carbon fiber sheet-like heat generating elements are disclosed in Japanese Patent Publication No.51-3098, Japanese Patent Publication No, 48-101634, Japanese Patent Laying-Open No.60-107288, Japanese Patent Laying-Open No.60-107289, Japanese Patent Laying-Open No.62-160681, Japanese Patent Laying-Open No.62-281293, Japanese Patent Laying-Open No. 63-19783, Japanese Patent Laying-Open No.1-112687, Japanese Patent Laying-Open No.5-13151, and Japanese Patent Laying-Open No.5-144554, for example.

Generally, in the carbon fiber sheet-like heat generating elements, the carbon fiber is formed into plane shape with shaping function material such as resin to stabilize the fiber structure. It is not originally preferred to use flammable materials such as resin because the carbon fiber sheet-like heat generating elements generate heat to a high temperature, but those are used unavoidably as plane stabilizing materials for stabilizing the carbon fiber in plane shape. Accordingly, flammable resins are not used as the plane stabilizing material, but resins having heat resistance are selected. The main applications of such carbon fiber sheet-like heat generating elements are heating, so that developments have been widely achieved with a primary view to its heat generating characteristics and electrical characteristics.

As described above, a lot of researches have been made so far on the carbon fiber sheet-like heat generating elements in the aspect of heat generation. However, researches have been hardly made in the aspect of the far-infrared radiators. Such far-infrared radiators are used not only for heating and warming but also used for the thermotherapy. According to the knowledge which the inventor of the present invention has acquired in his study, the far-infrared radiators can also be used for the purposes such as medical treatments which depend on the wavelengths of far-infrared rays. For that purpose, it is necessary to selectively and efficiently radiate the far-infrared rays in a particular wavelength range corresponding to the contents of the applications.

The inventor of the present invention has also acquired the information that there are far-infrared ray ranges which are peculiar to living bodies, organs or systems, although these are not detailed herein because the present invention is directed to a method and a structure for radiating the far-infrared rays.

Accordingly, the inventor of the present invention has proposed a far-infrared radiator and a method of radiating far-infrared rays using carbon fiber and organic compounds in Japanese Patent Laying-Open No.7-114974 and U.S. Patent 5,459,327. This far-infrared radiator and far-infrared ray radiating method can efficiently radiate far-infrared rays. The inventor then further studied and conducted experiments to still more efficiently radiate far-infrared rays.

Embodiments of the present invention can provide a far-infrared radiator and a method of radiating far-infrared rays which can still more efficiently radiate far-infrared rays in a desired particular wavelength range.

Embodiments of the invention can also provide a far-infrared radiator and a method of radiating far-infrared rays which can increase radiant energy of far-infrared rays at a long wavelength while suppressing an increase of radiant energy of infrared rays at a short wavelength.

As is stated in the above-cited Japanese Patent Laying-Open No.7-114974 and U.S. Patent 5,459,327, the inventor of the present invention paid attention to the carbon fiber as a far-infrared ray radiating material, not just as a mere heat generating element, but also to enable efficient radiation of far-infrared rays in a particular wavelength range. By "far-infrared rays" we mean infrared rays having wavelengths in the range of about 4 μm to about 100 μm (=1 mm).

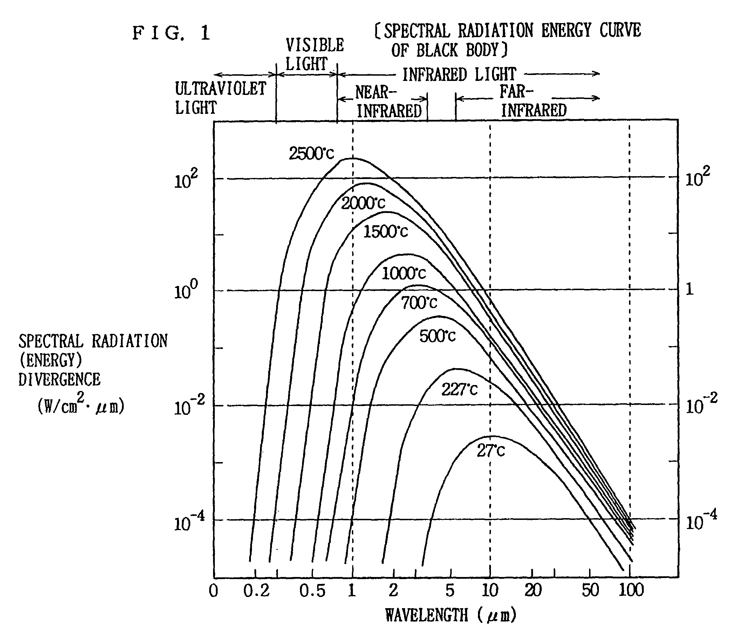

The spectral radiation energy curve of a black body is shown in Fig.1. The black body means an imaginary substance which absorbs all electromagnetic waves radiated from around over all wavelengths. The black body in the thermal equilibrium state with the outside about radiation emits all radiant energy received at that temperature to the outside. In other words, the black body has an absorptance of 1 and a reflectance of 0 at all wavelengths. On the other hand, real substances never absorb all of radiated energy by 100% like the black body and energy emitted therefrom is smaller than the received radiant energy. As a reference material, Theory and Practice of Far-infrared Heating (Ensekigai Kanetu No Riron To Jissai), edited by Japan Electro-heat Association, Long Wave Infrared Ray Subcommittee, OHMSHA, Japan, is cited herein, for example.

As can be seen from Fig.1, the peaks of the spectral radiation energy of the black body at various temperatures shift to shorter wavelengths as the temperature increases. The temperature of the black body having the peak in the wavelength range of the far-infrared rays (from 4 μm to 100 μm) ranges from the room temperature to about 200 °C at the most. Accordingly, high-temperature heating is not required to efficiently radiate the far-infrared rays.

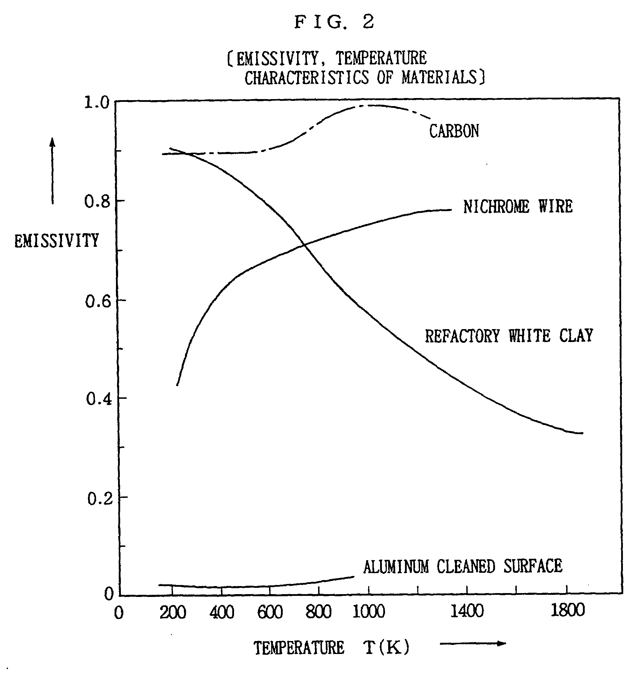

Fig.2 shows the temperature characteristics of emissivity of various materials. It can be seen from Fig.2 that the carbon has the largest emissivity in the range from 300 K (=27 °C) to 500 K (=227 °C). This emissivity of carbon is 0.9, which is very close to the emissivity of the black body, 1.0. This temperature range corresponds to the range in which the black body radiates the far-infrared rays most efficiently in the spectral radiation energy curve of the black body shown in Fig.1. From Fig.1 and Fig.2, it is understood that carbon radiates the far-infrared rays most efficiently. That is, materials other than carbon are not efficient as long as the radiation efficiency of the far-infrared rays only is considered.

Next, the inventor of the present invention paid attention to the infrared absorption spectrum of organic compounds, concerning selection of far-infrared rays in a particular wavelength range. Each organic compound shows infrared absorption spectrum which is peculiar to that organic compound corresponding to the natural frequency of its radical.

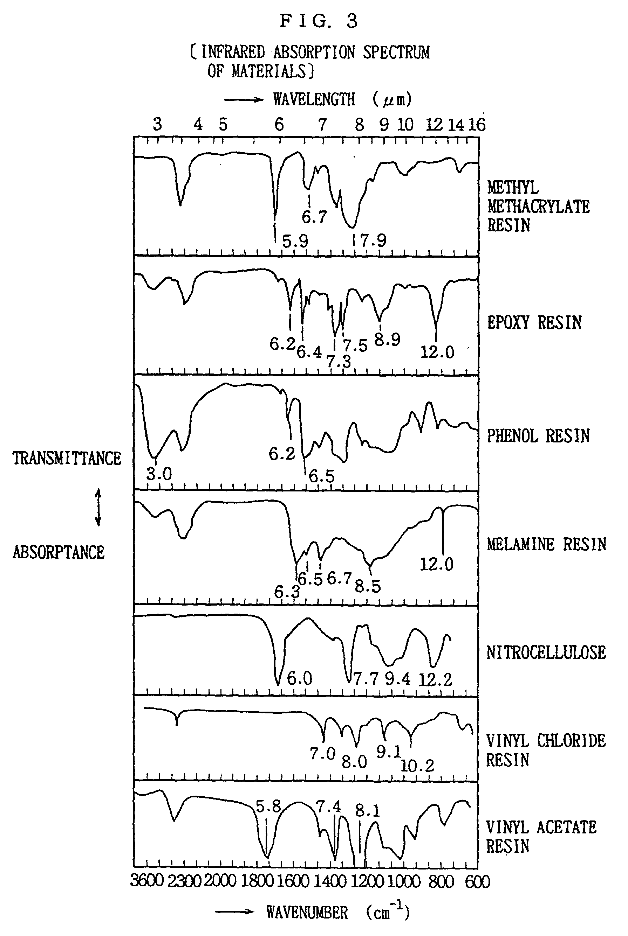

Fig.3 shows an example of the infrared absorption spectra of various resins. For example, methyl methacrylate resin has absorption peaks of far-infrared rays at the wavelengths 5.9 μm, 6.7 μm and 7.9 μm. The epoxy resin has absorption peaks of infrared rays at the wavelengths 6.2 μm, 6.4 μm, 7.3 μm, 7.5 μm, 8.9 μm and 12.0 μm. In this way, each kind of resin has its individual far-infrared absorption peaks.

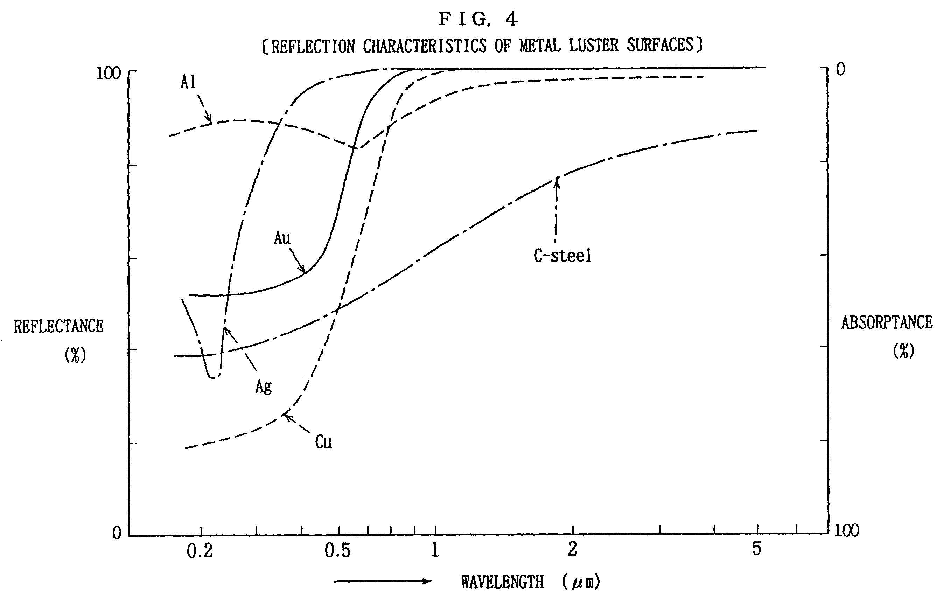

When an organic substance has absorbed far-infrared rays having a certain wavelength, resonance corresponding to the molecular state is caused inside the substance and far-infrared rays in a particular wavelength range are selectively radiated to the outside. That is to say, each substance has ability of radiating far-infrared rays in a wavelength range which depends on the wavelengths at which it has absorption peaks in the infrared absorption spectrum. Accordingly, far-infrared rays having desired wavelengths can be selected by using various kinds of organic compounds as a filter. On the other hand, as shown in Fig.4, metals such as aluminum, copper, silver and gold have reflectance of almost 100% in the wavelength range of the far-infrared rays. Accordingly, they can not be used as radiating elements for the far-infrared rays.

From the consideration made above, the inventor of the present invention has selected the carbon fiber as a material which most efficiently radiates the far-infrared rays, and studied to positively and efficiently use organic compounds for selecting far-infrared rays in a particular wavelength range.

Furthermore, the inventor paid attention to the fact that the far-infrared radiation from the carbon fiber, which is linear, is linear radiation and reached the idea that converting the linear radiation (line radiation) into surface radiation (area radiation) can further improve the radiant efficiency, and devised the present invention.

Now, although physical and physiological reasons of selecting the wavelengths of far-infrared rays lie in causing the far-infrared rays to molecular-resonate with the bio-structure and physical structure of the irradiated material to input useful energy, the principle thereof will not be described in the description of the present invention.

According to a method of radiating far-infrared rays of the present invention, far-infrared rays over a full far-infrared wavelength range are radiated by a heat generating element including carbon fiber mixed paper which is colored black, the radiated far-infrared rays are absorbed by a selected organic compound, and far-infrared rays in a certain wavelength range which depends on a molecular state in the selected organic compound are resonance-radiated.

The selected organic compound may include one or a plurality of resin layers laminated on the carbon fiber mixed paper. The one or plurality of resin layers may include a laminate of a plurality of kinds of resin layers.

According to a method of radiating far-infrared rays of another aspect of the present invention, far-infrared rays over a full far-infrared wavelength range are radiated by a heat generating element including carbon fiber, the radiated far-infrared rays are absorbed by a black-colored organic compound layer, and far-infrared rays in a certain wavelength range which depends on a molecular state in the organic compound layer are resonance-radiated from a surface of the organic compound layer, thereby converting the linear far-infrared radiation over the full far-infrared wavelength range into surface far-infrared radiation in the certain wavelength range.

The heat generating element may include carbon fiber mixed paper. The black-colored organic compound layer may include one or a plurality of resin layers laminated on the carbon fiber mixed paper.

According to a method of radiating far-infrared rays of still another aspect of the present invention, far-infrared rays over a full far-infrared wavelength range are radiated by a heat generating element including black-colored carbon fiber mixed paper, one or plural kinds of organic compounds are selected out of plural kinds of organic compounds, and the selected one or plural kinds of organic compounds absorb the radiated far-infrared rays and resonance-radiate far-infrared rays in a certain wavelength range which depends on a molecular state in the one or plural kinds of organic compounds selected.

The plural kinds of organic compounds may include a plurality of resin layers laminated on the carbon fiber mixed paper. The plurality of resin layers may include a plurality of kinds of resin layers arranged on the same plane.

According to a method of radiating far-infrared rays of still another aspect of the present invention, far-infrared rays over a full far-infrared wavelength range are radiated by a heat generating element including carbon fiber, one or a plurality of kinds of organic compound layers are selected out of a plurality of kinds of black-colored organic compound layers, the one or plurality of kinds of organic compound layers selected are caused to absorb the radiated far-infrared rays, and far-infrared rays in a certain wavelength range which depends on a molecular state in the one or plurality of kinds of organic compound layers selected are resonance-radiated from a surface of the organic compound layers, and thereby converting the linear far-infrared radiation over the full far-infrared wavelength range into surface far-infrared radiation in the certain wavelength range.

The heat generating element may include carbon fiber mixed paper. The plurality of kinds of organic compound layers may include a plurality of resin layers laminated on the carbon fiber mixed paper.

According to a far-infrared radiator of still another aspect of the present invention, electrodes are provided on carbon fiber mixed paper, the carbon fiber mixed paper being colored black, and a certain organic compound layer is laminated on the colored carbon fiber mixed paper.

In this case, a black substance may be impregnated in the carbon fiber mixed paper. Alternatively, a black substance may be applied or coated on the carbon fiber mixed paper. The organic compound layer may include one or a plurality of resin layers forming the carbon fiber mixed paper into sheet shape.

According to a far-infrared radiator of still another aspect of the present invention, electrodes are provided on carbon fiber mixed paper and a black-colored organic compound layer is laminated on the carbon fiber mixed paper.

In this case, a black substance may be mixed and dispersed in the organic compound layer. Alternatively, a black substance may be applied or coated on the organic compound layer. The organic compound layer may include a black-colored resin layer forming the carbon fiber mixed paper into sheet shape.

The carbon fiber mixed paper includes carbon fiber of a certain dimension dispersed in plant pulp composed of bast fiber.

In the far-infrared radiator and the far-infrared radiating method according to the present invention, the carbon fiber efficiently radiates far-infrared rays over the entire far-infrared wavelength range. At this time, since the carbon fiber is linear, the far-infrared radiation from the carbon fiber is linear radiation.

When the carbon fiber mixed paper is colored black, the far-infrared rays linearly radiated from the carbon fiber are efficiently absorbed into the entire surface of the carbon fiber mixed paper and far-infrared rays are radiated in plane from the surface of the carbon fiber mixed paper. Thus the group of linear radiation of far-infrared rays is converted into perfect surface radiation by the black carbon fiber mixed paper. That is to say, linear heat generation of the carbon fiber is converted into perfect surface heat generation.

Particularly, the efficiency of absorbing the far-infrared rays is high because the carbon fiber mixed paper is colored black, which slightly increases the rate of temperature rise of the carbon fiber mixed paper with respect to the supplied power. The radiant energy density E is shown by the equation:

$$E = kT^4.$$

Here T indicates an absolute temperature of the radiator and k is a certain coefficient. The change of the radiant energy density, $\Delta E$, is expressed by the following equation:

$$\Delta E = 4kT^3 \cdot \Delta T$$

Where $\Delta T$ indicates a change in absolute temperature of the radiator. From the equation above, it is seen that even a slight increase in temperature of the radiator considerably increases the radiant energy density.

The far-infrared rays radiated from the carbon fiber mixed paper are absorbed by a certain organic compound. Then the absorbed far-infrared rays resonate with molecular bond in that organic compound and far-infrared rays in a particular wavelength range are amplified and radiated from the organic compound. That is to say, the far-infrared rays in a large wavelength range absorbed into the organic compound are shifted and integrated in a particular wavelength range and converted into far-infrared rays in a small wavelength range. In this case, the energy in the large wavelength range is integrated into the small wavelength range and therefore the energy of the radiated far-infrared rays is amplified as compared with the energy in each absorbed wavelength range.

When the organic compound layer is colored black, the far-infrared rays linearly radiated from the carbon fiber are efficiently absorbed by the entire surface of the organic compound layer. In this case, the absorbed far-infrared rays also resonate with the molecular bond in the organic compound layer and far-infrared rays in a particular wavelength range are amplified and radiated in a plane from the surface of the organic compound layer. Thus the group of linear radiation of far-infrared rays is converted into perfect surface radiation by the black organic compound layer. That is to say, the linear heat generation of the carbon fiber is converted into perfect surface heat generation.

This way, the far-infrared linear radiation over the full far-infrared wavelength range is converted into far-infrared surface radiation in a particular wavelength range by the black carbon fiber mixed paper or by the black organic compound layer. The rate of temperature increase of the carbon fiber mixed paper or the organic compound layer with respect to the supplied power is slightly increased. This enables far-infrared rays in a particular wavelength range to be radiated with a high radiant energy density. Furthermore, the high radiant energy density can be obtained with small power consumption, leading to energy saving.

Thus, according to the far-infrared radiator and the far-infrared ray radiating method of the present invention, far-infrared rays in a desired particular wavelength range can be radiated at a high efficiency.

Also, since the temperature of the carbon fiber mixed paper only slightly increases, the wavelength of the radiated far-infrared rays is not shifted to shorter wavelengths. (Refer to Fig.1.) That is to say, it is possible to increase the radiant energy of far-infrared rays at long wavelength while suppressing an increase of radiant energy of infrared rays at short wavelength.

While the short-wavelength infrared rays warm air, the long-wavelength far-infrared rays directly vibrate molecules of water ($H_2O$) and carbon dioxide ($CO_2$) of a living body without warming the air almost at all. Hence, it is possible to directly heat or warm a living body with small power consumption, leading to energy saving.

According to the far-infrared radiator and the far-infrared ray radiating method of the present invention, it is also possible to radiate far-infrared rays in a desired wavelength range even at a temperature of 0°C or lower. This will be very helpful to give heat to specified materials without heating other environmental molecules like air.

The foregoing and other objects, features, aspects and advantages of the present invention will become more apparent from the following detailed description of embodiments of the present invention when taken in conjunction with the accompanying drawings, in which:

Fig.1 is a diagram showing a spectral radiation energy curve of a black body.

Fig.2 is a diagram showing temperature characteristics of emissivity of various kinds of materials.

Fig.3 is a diagram showing the infrared absorption spectra of various kinds of resins.

Fig.4 is a diagram showing the reflection characteristics of various kinds of metal luster surfaces.

Fig.5A is a plane view of a far-infrared radiator according to a first embodiment of the present invention.

Fig.5B is a sectional view of an end portion of the far-infrared radiator according to the first embodiment of the present invention.

Fig.6 is a sectional view showing another example of structure of the organic compound layer in the far-infrared radiator shown in Fig.5.

Fig.7 is a sectional view of a far-infrared radiator according to a second embodiment of the present invention.

Fig.8 is a sectional view of a far-infrared radiator according to a third embodiment of the present invention.

Fig.9 is a plan of a far-infrared radiator according to a fourth embodiment of the present invention.

Fig.10 is a sectional view of the far-infrared radiator of Fig.9.

Fig.11A is a plan of a far-infrared radiator according to a fifth embodiment of the present invention.

Fig.11B is a sectional view of an end of the far-infrared radiator of Fig.11A.

Fig.12 is a sectional view of a far-infrared radiator according to a sixth embodiment of the present invention.

Fig.13 is a diagram showing a method of measuring amounts of spectral radiation energy of far-infrared radiators of the invention and a comparison example.

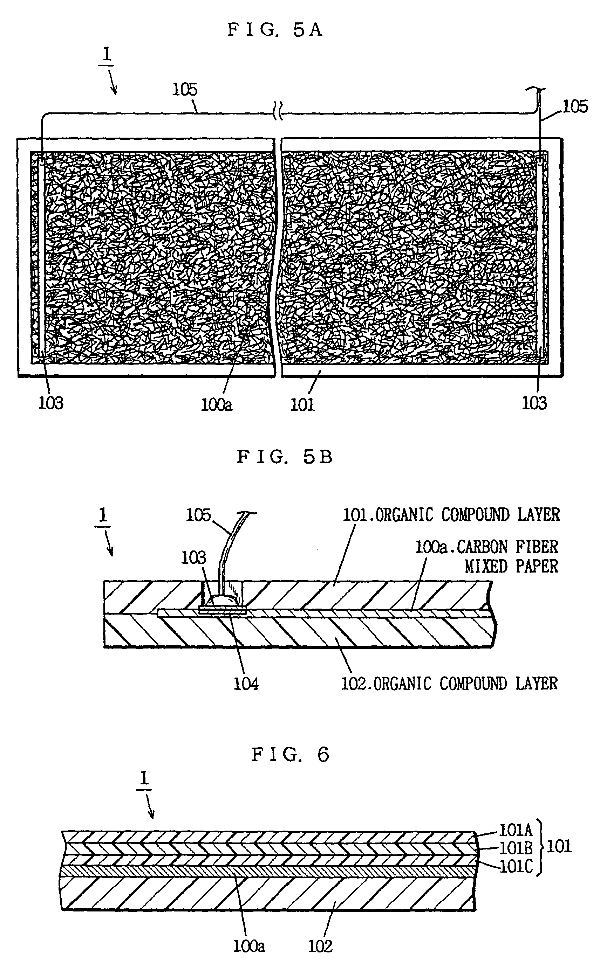

Fig.5A is a plan of a far-infrared radiator according to a first embodiment of the present invention and Fig.5B is a sectional view of an end portion of the far-infrared radiator. This far-infrared radiator 1 includes carbon fiber mixed paper 100a which is colored black and organic compound layers 101 and 102 laminated on both sides of the carbon fiber mixed paper 100a.

The carbon fiber mixed paper is produced as described below. A pulp liquid is made by adding water to bast fiber such as the paper mulberry, the mitsumata (*Edgeworthia papyrifera*), or the ganpi (*Wikstroemia sikokiana*) which are used as materials of Japanese paper. Then, carbon fiber which is cut to about 5 mm is mixed therein and dispersed. The pulp liquid is supplied on a net for paper making to form a wet sheet. The wet sheet is mechanically dehydrated using a roll for water squeeze and dried, and then cut to predetermined dimensions. In this way, the carbon fiber mixed paper having a thickness of about 0.1 mm is formed.

As materials of the organic compound layers 101 and 102, thermosetting resins or thermoplastic resins are used, for example. The thermosetting resins include phenol resin, melamine resin, furan resin, unsaturated polyester resin, diallyl phthalate resin, epoxy resin, silicone resin, polyimide resin, urethane resin and so forth. The thermoplastic resins include vinyl chloride resin, vinyl acetate resin, vinylidene chloride resin, polystyrene, acrylonitrile-styrene resin, acrylonitrile-butadiene-styrene resin, methyl methacrylate resin, ethylene-vinyl acetate resin, polyamide, polyimide, polyamideimide, polyurethane, polycarbonate, polyester, nitrocellulose and so forth. Organic compounds containing heavy metal elements such as iron, copper, silver and platinum can also be used as the organic compound layers 101 and 102. As described above, as to the organic compound layers 101 and 102, any materials having absorption peaks in the wavelength range of the far-infrared rays can be used for the purpose of the present invention by evaluating the peaks.

The materials of the organic compound layers 101 and 102 are selected corresponding to the wavelength range of radiated far-infrared rays. It is not necessary that the material of the organic compound layer 101 is the same as the material of the organic compound layer 102, which may be composed of different materials. In this case, far-infrared rays in different wavelength ranges are radiated from the front surface and the back surface of the far-infrared radiator 1.

Furthermore, the thermal resistance and combustibility of the organic compound layers 101 and 102 may be left out of consideration because the far-infrared radiator 1 of this embodiment is used to efficiently radiate far-infrared rays in a particular wavelength range.

Now, a method of producing the far-infrared radiator 1 shown in Fig.5A and Fig.5B will be described in an example in which the organic compound layers 101 and 102 are formed of fiber-glass reinforced epoxy resin (glass-epoxy resin).

Strip-like silver paste 104 is printed along opposite two sides of the carbon fiber mixed paper and electrodes 103 composed of copper-foil tape with electrically conductive adhesive applied thereon are bonded on the silver paste 104. Then a black substance such as black paint, e.g., CuO (copper oxide), $Fe_3O_4$ (iron(II) iron(III) oxide or ferrosoferric oxide), $Fe_3P$ (triiron monophosphide), $Fe_2MgO_4$, (magnesium iron oxide or magnesium ferrite), $Fe(C_9H_7)_2$ (bis (indenyl) iron), is applied (coated) or impregnated to the carbon fiber mixed paper to produce the black carbon fiber mixed paper 100a. The carbon fiber mixed paper may be colored black before the pair of electrodes 103 are attached to the carbon fiber mixed paper. Or, in the process of manufacturing the carbon fiber mixed paper, the black carbon fiber mixed paper 100a may be manufactured by mixing and dispersing a black substance such as black pigment in pulp liquid.

Then, the carbon fiber mixed paper 100a is sandwiched between damp-dried glass-epoxy resin layers and hot-pressed to thermally harden the glass-epoxy resin layers. At this time, as shown in Fig.5B, a hole for provision of lead wire is formed in the glass-epoxy resin layer in the area at the end of each electrode 103.

As described above, the organic compound layers 101 and 102 composed of glass-epoxy resin are laminated on both sides of the black carbon fiber mixed paper 100a. Finally, the lead wire 105 is connected to one end of each electrode 103 by soldering, or the like. A far-infrared radiator 1 having a thickness of about 0.5 mm is thus produced.

When a voltage is applied to the electrodes 103 of the far-infrared radiator 1 through the lead wires 105, a current flows to the carbon fiber dispersed in the carbon fiber mixed paper 100a. The carbon fiber then generates heat and radiates far-infrared rays. The far-infrared rays radiated from the carbon fiber are absorbed by the black carbon fiber mixed paper 100a and radiated in a plane from the front and back surfaces of the carbon fiber mixed paper 100a. The linear far-infrared radiation from the carbon fiber is thus converted into surface radiation by the black carbon fiber mixed paper 100a. In other words, the linear heat generation of the carbon fiber is converted into complete surface heat generation.

When the organic compound layers 101 and 102 absorb the far-infrared rays at the infrared absorption peaks, resonance is caused inside the organic compound layers 101 and 102 and far-infrared rays in a particular wavelength range are selectively radiated to the outside. That is to say, the organic compound layers 101 and 102 have ability of converting received heating energy into far-infrared rays in a particular wavelength range and radiating them.

By forming the organic compound layers 101 and 102 with various organic compounds, far-infrared rays in various wavelength ranges are efficiently radiated depending on the wavelengths of the infrared absorption peaks of those organic compounds. That is to say, the far-infrared radiator 1 has wavelength selectivity which depends on the kinds of the organic compound layers 101 and 102.

This way, in the far-infrared radiator 1 of this embodiment, the linear far-infrared radiation from the carbon fiber is converted into surface radiation by the black carbon fiber mixed paper 100a, which increases the radiant efficiency of the far-infrared rays. This allows the far-infrared rays in the wavelength range which depends on the kinds of the organic compound layers 101, 102 to be radiated at a high efficiency (with high energy density).

Each of the organic compound layers 101 and 102 can be formed of laminated plural kinds of organic compounds as needed. In the example shown in Fig.6, the organic compound layer 101 includes a laminate of three kinds of organic compound layers 101A, 101B and 101C.

In this case, the infrared absorption peaks of the three kinds of organic compound layers 101A, 101B and 101C are compounded and far-infrared rays in a wavelength range corresponding to the compounded infrared absorption peak are selectively radiated.

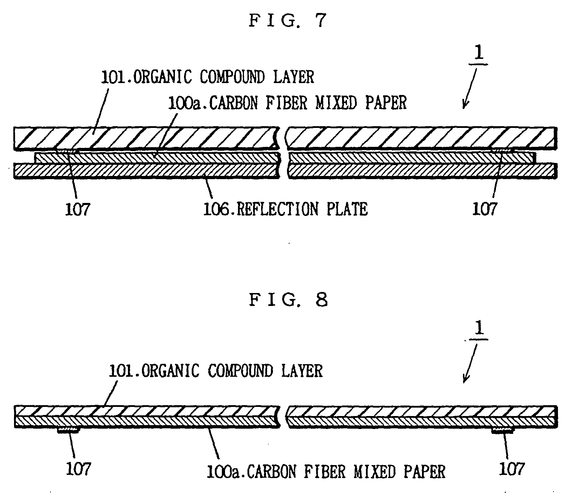

Fig.7 is a sectional view of a far-infrared radiator according to a second embodiment of the present invention. This far-infrared radiator 1 includes black-colored carbon fiber mixed paper 100a, an organic compound layer 101 and a reflection plate 106.

The back surface of the carbon fiber mixed paper 100a is bonded on the reflection plate 106. A pair of electrodes 107 are formed along the opposite two sides on the surface of the carbon fiber mixed paper 100a. The organic compound layer 101 is exchangeably provided on the carbon fiber mixed paper 100a.

The carbon fiber mixed paper 100a and the organic compound 101 are the same as the carbon fiber mixed paper 100a and the organic compound layer 101 in the first embodiment, respectively. The reflection plate 106 is composed of a metal plate, such as aluminum, copper, silver and gold, or a metal-coated plate. These metals have reflectance to almost 100 % in the wavelength range of the far-infrared rays as shown in Fig.4. Accordingly, most of all the far-infrared rays radiated from the back surface of the carbon fiber mixed paper 100a are reflected in the direction to the organic compound layer 101 by the reflection plate 106. This improves the efficiency of radiation of the far-infrared rays.

In the far-infrared radiator 1 according to this embodiment, the wavelength range of the far-infrared rays to be radiated can be easily controlled by changing the organic compound layer 101 for an organic compound layer made of another material.

Fig.8 is a sectional view of a far-infrared radiator according to a third embodiment of the present invention. This far-infrared radiator 1 includes black-colored carbon fiber mixed paper 100a and an organic compound layer 101 coated on the surface of the carbon fiber mixed paper 100a.

An organic compound which is dissolvable in a solvent is used as the organic compound layer 101. This organic compound layer 101 is formed by applying or coating an organic compound dissolved in a solvent on the surface of the carbon-fiber mixed paper 100a. A pair of electrodes 107 are formed along the opposite two sides on the back surface of the carbon fiber mixed paper 100a.

In the far-infrared radiator 1 of this embodiment, the far-infrared rays in a wavelength range which depends on the kind of the organic compound 101 coated on the surface of the carbon fiber mixed paper 100a are selectively and efficiently radiated, too.

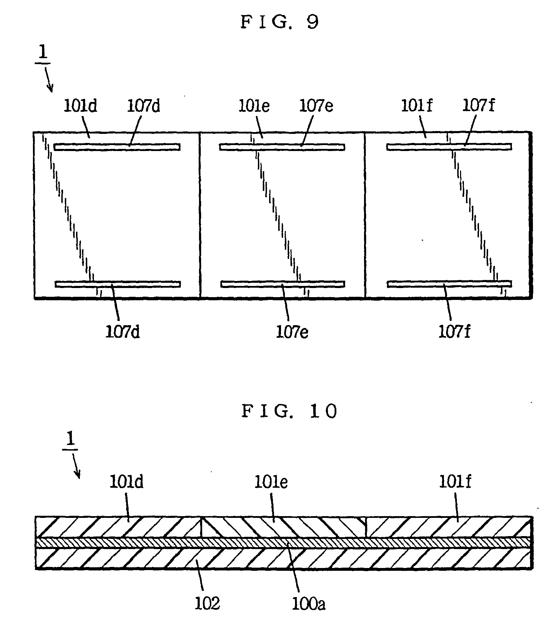

Fig.9 is a plane view of a far-infrared radiator according to a fourth embodiment of the present invention and Fig.10 is a sectional view of the far-infrared radiator. This far-infrared radiator 1 includes black-colored carbon fiber mixed paper 100a, three kinds of organic compound layers 101d, 101e and 101f laminated on the surface of the carbon fiber mixed paper 100a and an organic compound layer 102 laminated on the back surface of the carbon fiber mixed paper 100a. The organic compound layers 101d, 101e and 101f are composed of different materials.

On the surface of the carbon fiber mixed paper 100a, a pair of electrodes 107d are formed in the area corresponding to the organic compound layer 101d, a pair of electrodes 107e are formed in the area corresponding to the organic compound layer 101e and a pair of electrodes 107f are formed in the area corresponding to the organic compound layer 101f.

In the far-infrared radiator of this embodiment, by applying current to one of the three pairs of electrodes 107d, 107e and 107f, far-infrared rays in a wavelength range which depends on the kind of the corresponding organic compound layer are selectively and efficiently radiated.

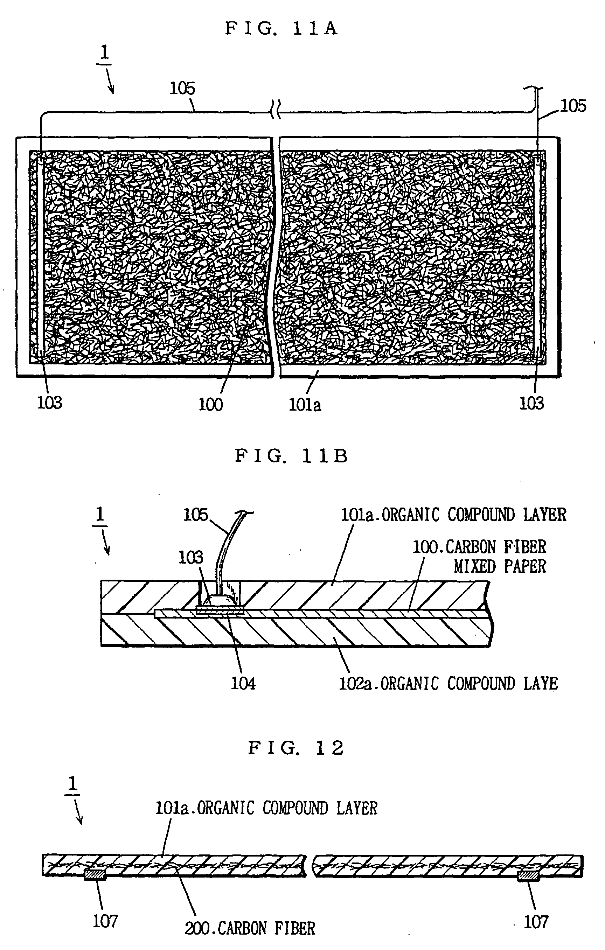

Fig.11A is a plan of a far-infrared radiator according to a fifth embodiment of the present invention and Fig.11B is a sectional view of an end of the far-infrared radiator. This far-infrared radiator 1 includes carbon fiber mixed paper 100 which is not colored and black organic compound layers 101a and 102a laminated on both sides of the carbon fiber mixed paper 100.

The far-infrared radiator 1 of this embodiment differs from the far-infrared radiator 1 shown in Fig.5 in that the uncolored carbon fiber mixed paper 100 is used instead of the black-colored carbon fiber mixed paper 100a, and that the organic compound layers 101a and 102a which are colored black are used instead of the uncolored organic compound layers 101 and 102. In other respects, the structure of the far-infrared radiator 1 of this embodiment is the same as the structure of the far-infrared radiator of the first embodiment.

The method of manufacturing the carbon fiber mixed paper 100 is the same as the method of manufacturing the carbon fiber mixed paper 100a shown in Fig.5 except that it is not colored. Materials of the organic compound layers 101a and 102a are the same as those of the organic compound layers 101 and 102 in the first embodiment. However, the organic compound layers 101a and 102a of this embodiment may be formed by mixing and dispersing a black substance such as black pigment, e.g., carbon black, $CuO$, $Fe_3O_4$, $Fe_3P$, $Fe_2MgO_4$, $Fe(C_9H_7)_2$, into an organic compound material, or they may be formed by applying or coating a black substance such as black paint on the surfaces of uncolored organic compound layers.

When a voltage is applied to the electrode 103 of the far-infrared radiator 1 through the lead wire 105, a current flows in the carbon fiber dispersed in the carbon fiber mixed paper 100 and then the carbon fiber generates heat and radiates far-infrared rays. When the black organic compound layers 101a and 102a absorb the far-infrared rays at the wavelength of the infrared absorption peak, resonance occurs in the organic compound layers 101a and 102a and far-infrared rays in a particular wavelength range are radiated in a plane from the front surface and the back surface of the organic compound layers 101a and 102a. Thus the linear radiation of the far-infrared rays from the carbon fiber is converted into perfect surface radiation by the black organic compound layers 101a and 102a. In other words, the linear heat generation is converted into perfect surface heat generation.

This way, the organic compound layers 101a and 102a have ability of converting the linear far-infrared radiation into surface radiation and converting received heat generation energy into far-infrared rays in a particular wavelength range and radiating them.

In the embodiment, forming the organic compound layers 101a and 102a with various organic compounds also allows far-infrared rays in various wavelength ranges depending on the wavelengths of infrared absorption peaks of the organic compounds to be efficiently radiated.

Fig.12 is a sectional view of a far-infrared radiator according to a sixth embodiment of the present invention. This far-infrared radiator 1 includes a black-colored organic compound layer 101a and carbon fiber 200 mixed and dispersed in the organic compound layer 101a. The far-infrared radiator 1 is manufactured by mixing and dispersing carbon fiber and a black substance such as black pigment, CuO, for example, into a liquid organic compound and then forming the organic compound into sheet shape. At this time, a pair of electrodes 107 are formed in contact with the carbon fiber 200 on the back of the organic compound layer 101a.

The far-infrared radiator 1 of this embodiment also allows the far-infrared rays in wavelength ranges which depend on the kinds of the organic compound layer 101a to be selectively and efficiently radiated.

In the example shown in Fig.6, carbon fiber mixed paper which is not colored may be used in place of the black carbon fiber mixed paper 100a and a laminate structure of black organic compound layers and a black organic compound layer may be used in place of the uncolored organic compound layers 101A, 101B, 101C and the organic compound layer 102.

in the embodiments of Fig.7 and Fig.8, uncolored carbon fiber mixed paper may be used in place of the black carbon fiber mixed paper 100a and a black organic compound layer may be used in place of the organic compound layer 101 which is not colored.

Furthermore, in the embodiments of Fig.9 and Fig.10, uncolored carbon fiber mixed paper may be used in place of the black carbon fiber mixed paper 100a and black organic compound layers may be used in place of the uncolored organic compound layers 101d, 101e, 101f and the organic compound layer 102.

Alternatively, the far-infrared radiator 1 may be formed of black carbon fiber mixed paper and a black organic compound layer.

Now, we manufactured the far-infrared radiator of the fifth embodiment shown in Fig.11A and Fig.11B and a far-infrared radiator for a comparison example and measured amounts of the spectral radiation energy. In the far-infrared radiator of the embodiment, the organic compound layers 101a, 102a were formed of epoxy resin containing a black glass cloth base (a highly heat-resistant epoxy resin black glass base laminate). In the far-infrared radiator of the comparison example, organic compound layers were formed of epoxy resin containing an uncolored glass cloth base (a highly heat-resistant epoxy resin glass base laminate). The structure of other parts of the far-infrared radiator of the comparison example were the same as those of the far-infrared radiator of the embodiment.

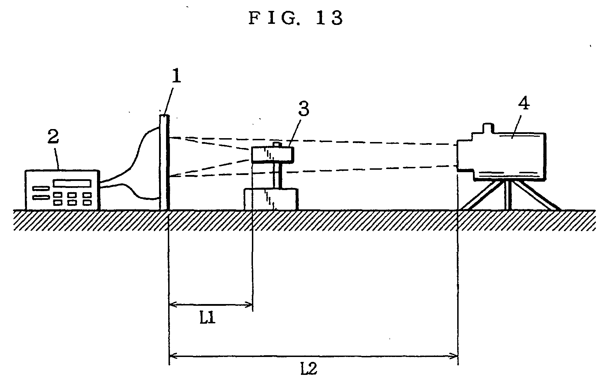

Fig.13 shows the method of measuring the amounts of spectral radiation energy. As shown in Fig.13, the far-infrared radiator 1 was disposed vertically to the floor. The outside dimensions of the far-infrared radiators 1 of the embodiment and the comparison were both 250mm×250mm. An integrating wattmeter 2 was connected to the far-infrared radiator 1. A radiation thermometer 3 was disposed in front of the far-infrared radiator 1 and an infrared spectral energy measuring device (infrared spectrophotometer) 4 was disposed further ahead. The distance L1 from the surface of the far-infrared radiator 1 to the radiation thermometer 3 was 300mm and the measured area on the far-infrared radiator 1 by the radiation thermometer 3 was an area of 118 mm in diameter. The distance L2 from the surface of the far-infrared radiator 1 to the infrared spectral energy measuring device 4 was 1000 mm and the measured area on the far-infrared radiator 1 by the infrared spectral energy measuring device 4 was an area with a diameter of 118 mm.

An integrating wattmeter of type 2534 produced by Yokokawa Electric Corporation was used as the integrating wattmeter 2, a radiation thermometer of type IT-340 produced by Horiba, Ltd. was used as the radiation thermometer 3 and a trade name "Thermolex", type TMM-P-10 produced by Kabushiki Kaisha Thermomic was used as the infrared spectral energy measuring device 4.

With the far-infrared radiator 1 supplied with power, the amounts of spectral radiation energy in each wavelength region were measured by the infrared spectral energy measuring device 4 and the surface temperature of the far-infrared radiator 1 was measured by the radiation thermometer 3. The measurements were conducted five times. The emissivity e of the radiation thermometer 3 was 1.0. The power consumption of the far-infrared radiator 1 was measured by the integrating wattmeter 2. The power consumption of the far-infrared radiator 1 was 45 W (power density =0.093[W/cm$^2$]).

Table 1 shows measurements of amounts of spectral radiation energy of the far-infrared radiator of the embodiment and Table 2 shows the measurements of the amounts of spectral radiation energy of the far-infrared radiator of the comparison example.

Table 1

| | Embodiment | | | |
|---|---|---|---|---|
| Number of measurements | Surface temperature(°C) | Amount of spectral radiation energy in each wavelength region ($\times 10^{-3}$W/cm$^2$) | | |
| | | F1 | F2 | F3 |
| 1st time | 64.6 | 0.08 | 6.24 | 15.2 |
| 2nd time | 64.2 | 0.09 | 6.21 | 15.1 |
| 3rd time | 64.0 | 0.09 | 6.28 | 15.3 |
| 4th time | 64.3 | 0.09 | 6.16 | 15.1 |
| 5th time | 62.8 | 0.09 | 6.24 | 15.3 |
| Average | 64.0 | 0.09 | 6.23 | 15.2 |

Table 2

| | Comparison example | | | |
|---|---|---|---|---|
| Number of measurements | Surface temperature(°C) | Amount of spectral radiation energy in each wavelength region ($\times 10^{-3}$W/cm$^2$) | | |
| | | F1 | F2 | F3 |
| 1st time | 62.0 | 0.07 | 5.42 | 13.8 |
| 2nd time | 62.2 | 0.07 | 5.48 | 13.8 |
| 3rd time | 62.5 | 0.08 | 5.56 | 14.0 |
| 4th time | 62.0 | 0.07 | 5.43 | 13.8 |
| 5th time | 62.3 | 0.08 | 5.46 | 13.8 |
| Average | 62.2 | 0.07 | 5.47 | 13.8 |

In Table 1 and Table 2, F1, F2 and F3 show the wavelength region of 0.7 to 3 μm, the wavelength region of 3 to 7 μm and the wavelength region of 7 to 14 μm, respectively.

From Table 1 and Table 2, it is seen that the surface temperature of the far-infrared radiator of the embodiment is higher by about 1.8 degrees than the far-infrared radiator of the comparison example and the amount of spectral radiation energy is larger by 10 to 30%. Particularly, in the far-infrared radiator of the embodiment, large amounts of spectral radiation energy were obtained in the wavelength region of 3 to 7 μm and the wavelength region of 7 to 14 μm included in the far-infrared wavelength range. It is thus seen that laminating black organic compound layers on carbon fiber mixed paper increases the amount of radiant energy in the far-infrared wavelength range.

This way, the use of the far-infrared radiator 1 of the embodiment increases the amount of radiant energy without an increase of supplied power. Furthermore, the slight increase in surface temperature of the far-infrared radiator 1 does not cause the wavelength of the radiated far-infrared rays to shift to shorter wavelengths. Accordingly, it is possible to increase the amount of radiant energy in the far-infrared wavelength range without increasing the power consumption.

Coloring the carbon fiber mixed paper black instead of coloring the organic compound layers black also increases the amount of radiant energy in the far-infrared wavelength range.

The far-infrared radiator 1 of the above-described embodiments can be used for medical treatments to various parts of a living body. When the wavelength range of far-infrared rays to be radiated differs depending on the position on the living body or the contents of the treatments, the kinds of the organic compound layers can be selected so that far-infrared rays in a wavelength range which is the most suitable for that part or the contents of the treatment are radiated. Thus, various parts of a living body can be treated with the far-infrared rays in the most suitable wavelength ranges.

Furthermore, the far-infrared radiator 1 of the above-described embodiments can be effectively used for radiant heating and radiant warming. In the conventional study of sheet-like heat generators, attentions have been paid only to the temperature rise of the sheet-like heat generator itself. That is to say, in the conventional heating, the surface temperature of a heater was increased to increase the temperature of air in a room.

However, the far-infrared radiator 1 of the embodiments can increase the amount of radiant energy of the far-infrared rays while hardly increasing the surface temperature, as stated above, so that the wavelength of the radiated far-infrared rays is not shifted to shorter wavelengths. The infrared rays at a short wavelength warm air but the far-infrared rays at a long wavelength affect molecules of water ($H_2O$) and molecules of carbon dioxide ($CO_2$) while hardly affecting the air. Accordingly, the radiant energy radiated from the far-infrared radiator 1 goes straight through the air to reach an object, where most of it is absorbed. This activates motion of the molecules inside the object to increase the temperature.

In this way, an object is directly heated by the radiated far-infrared rays in the heating and warming using the far-infrared radiator 1. Accordingly, the far-infrared radiator 1 can work not only on living bodies but also on all substances which require far-infrared radiation.

From the reasons mentioned above, unlike the heating which warms air, the temperature rise of the heat generator itself is not a problem but the increase of the radiation temperature is important in the heating and warming by the far-infrared radiation. Hence, the use of the far-infrared radiators 1 of the above-described embodiments enables efficient heating or warming, leading to energy conservation.

While the invention has been described in detail, the foregoing description is in all aspects illustrative and not restrictive. It is understood that numerous other modifications and variations can be devised without departing from the scope of the invention.

### Claims

1. A method of generating far-infrared radiation, comprising generating far-infrared rays over a full far-infrared wavelength range with a heat generating element including carbon fiber mixed paper (100a), and causing a selected organic compound to absorb said far-infrared radiation and to resonance-radiate far-infrared radiation in a selected wavelength range which depends on a molecular state in said selected organic compound, **characterised in that** the carbon fiber mixed paper (100a) is coloured black by impregnating a black substance in the carbon fiber mixed paper or by applying or coating a black substance on said carbon fiber mixed paper.

2. A method according to claim 1, wherein said selected organic compound is present as an organic compound layer (101,102) and said layer includes one or a plurality of resin layers (101A,B,C,d,e,f) laminated on said carbon fiber mixed paper (100a).

3. A method according to claim 2, wherein said one or plurality of resin layers include a laminate of a plurality of kinds of resin layers (101A,B,C).

4. A method according to claim 1, wherein a plurality of different organic compounds are caused to absorb said far-infrared radiation and to resonance-radiate far-infrared radiation in respective selected wavelength ranges.

5. A method according to claim 4, wherein the plurality of different organic compounds are present as a plurality of layers, said layers comprising a plurality of resin layers (101A,B,C,d,e,f) laminated on said carbon fiber mixed paper.

6. A method according to claim 5, wherein the plurality of resin layers include a plurality of kinds of resin layers (101d, e,f) arranged on the same plane.

7. A method of generating far-infrared radiation, comprising generating far-infrared rays over a full far-infrared wavelength range with a heat generating element including carbon fiber (100,200), and causing an organic compound layer (101a,102a) to absorb said radiated far-infrared rays and to resonance-radiate far-infrared rays in a selected wavelength range which depends on a molecular state in said organic compound layer (101,102a) from a surface of said organic compound layer, **characterised in that** the organic compound layer is coloured black by mixing and dispersing a black substance in the organic compound layer or by applying or coating a black substance on the organic compound layer.

8. A method according to claim 7, wherein said heat generating element includes carbon fiber mixed paper (100).

9. A method according to claim 8, wherein the black-colored organic compound layer (101a,102a) includes one or a plurality of resin layers laminated on said carbon fiber mixed paper.

10. A far-infrared radiator (1), comprising:

(a) a heat generating element including carbon fiber mixed paper (100a), wherein electrodes (103,107) are provided on the carbon fiber mixed paper; and
(b) an organic compound layer (101,102) laminated on said carbon fiber mixed paper,

**characterised in that** the carbon fiber mixed paper (100a) is coloured black.

11. A far-infrared radiator according to claim 10, wherein a black substance is impregnated in said carbon fiber mixed paper (100a) to form the black coloured carbon fiber mixed paper.

12. A far-infrared radiator according to claim 10, wherein a black substance is applied on said carbon fiber mixed paper (100a) to form the black coloured carbon fiber mixed paper.

13. A far-infrared radiator according to any one of claim 10 to 12, wherein said organic compound layer (101,102) includes one or a plurality of resin layers (101A,B,C,d,e,f) to form said carbon fiber mixed paper into sheet shape.

14. A far-infrared radiator (1), comprising:

(a) a heat generating element including carbon fiber mixed paper (100), wherein electrodes (103) are provided on the carbon fiber mixed paper; and
(b) an organic compound layer (101a,102a) laminated on said carbon fiber mixed paper,

**characterised in that** the organic compound layer (101a,102a) is black coloured.

15. A far-infrared radiator according to claim 14, wherein a black substance is mixed and dispersed in said organic compound layer (101a,102a) to form the black coloured organic compound layer.

16. A far-infrared radiator according to claim 14, wherein a black substance is applied on said organic compound layer (101a,102a) to form the black coloured organic compound layer.

17. A far-infrared radiator according to any one of claims 14 to 16, wherein said organic compound layer (101a,102a) includes a black-colored resin layer forming said carbon fiber mixed paper into sheet shape.

**Patentansprüche**

1. Verfahren zur Erzeugung von Strahlung im fernen Infrarot, umfassend das Erzeugen von Strahlen im fernen Infrarot über einen vollen Wellenlängenbereich im fernen Infrarot mit einem wärmeerzeugenden Element, das mit Kohlenstofffasern gemischtes Papier (100a) enthält, und das Veranlassen einer ausgewählten organischen Verbindung zur Absorption der Strahlung im fernen Infrarot und zur Resonanz-Abstrahlung der Strahlung im fernen Infrarot in einem ausgewählten Wellenlängenbereich, der von einem Molekülzustand in der ausgewählten organischen Verbindung abhängt, **dadurch gekennzeichnet, dass** das mit Kohlenstofffasern gemischte Papier (100a) schwarz gefärbt wird, indem das mit Kohlenstofffasern gemischte Papier mit einer schwarzen Substanz imprägniert wird oder indem eine schwarze Substanz auf das mit Kohlenstofffasern gemischte Papier aufgebracht oder aufgetragen wird.

2. Verfahren nach Anspruch 1, worin die ausgewählte organische Verbindung als Schicht aus einer organischen Verbindung (101, 102) vorliegt und die Schicht eine oder eine Vielzahl von Harzschichten (101A,B,C,d,e,f) umfasst, die auf das mit Kohlenstofffasern gemischte Papier (100a) auflaminiert wird.

3. Verfahren nach Anspruch 2, worin die eine oder die Vielzahl von Harzschichten ein Laminat aus einer Vielzahl von Arten von Harzschichten (1001A,B,C) umfasst.

4. Verfahren nach Anspruch 1, worin eine Vielzahl verschiedener organischer Verbindungen zur Absorption der Strahlung im fernen Infrarot und zur Resonanz-Abstrahlung der Strahlung im fernen Infrarot in jeweiligen ausgewählten Wetlenlängenbereichen veranlasst wird.

5. Verfahren nach Anspruch 4, worin die Vielzahl verschiedener organischer Verbindungen als eine Vielzahl von Schichten vorhanden ist, wobei die Schichten eine Vielzahl von Harzschichten (101A,B,C,d,e,f) umfasst, die auf das mit Kohlenstofffäsern gemischte Papier auflaminiert sind.

6. Verfahren nach Anspruch 5, worin die Vielzahl von Harzschichten eine Vielzahl von Arten von Harzschichten (101d, e,f) umfasst, die in der selben Ebene angeordnet sind.

7. Verfahren zur Erzeugung von Strahlung im fernen Infrarot, umfassend das Erzeugen von Strahlen im fernen Infrarot über einen vollen Wellenlängenbereich im fernen Infrarot mit einem wärmeerzeugenden Element, das Kohlenstofffasern (100, 200) enthält, und das Veranlassen einer Schicht aus einer organischen Verbindung (101a, 102a) zur Absorption der ausgesandten Strahlen im fernen Infrarot und zur Resonanz-Abstrahlung von Strahlen im fernen Infrarot in einem ausgewählten Wellenlängenbereich, der von einem Molekülzustand in der Schicht aus der organischen Verbindung (101a, 102a), von einer Oberfläche der Schicht aus der organischen Verbindung abhängt, **dadurch gekennzeichnet, dass** die Schicht aus der organischen Verbindung schwarz gefärbt wird, indem eine schwarze Substanz in die Schicht aus der organischen Verbindung gemischt oder darin dispergiert wird oder indem ein schwarzes Substrat auf die Schicht aus der organischen Verbindung aufgebracht oder aufgetragen wird.

8. Verfahren nach Anspruch 7, worin das wärmeerzeugende Element mit Kohlenstofffasern gemischtes Papier (100) umfasst.

9. Verfahren nach Anspruch 8, worin die schwarz gefärbte Schicht aus der organischen Verbindung (101a, 102a) eine oder eine Vielzahl von Harzschichten umfasst, die auf das mit Kohlenstofffasern gemischte Papier auflaminiert ist.

10. Fern-Infrarot-Strahler, umfassend:

    (a) ein wärmeerzeugendes Element, das mit Kohlenstofffasern gemischtes Papier (100a) umfasst, worin Elektroden (103, 107) auf dem mit Kohlenstofffasern gemischten Papier vorgesehen sind; und
    (b) eine Schicht aus einer organischen Verbindung (101, 102), die auf das mit Kohlenstofffasern gemischte Papier auflaminiert ist,

    **dadurch gekennzeichnet, dass** das mit Kohlenstofffasern gemischte Papier (100a) schwarz gefärbt ist.

11. Fern-Infrarot-Strahler nach Anspruch 10, worin das mit Kohlenstofffasern gemischte Papier (100a) mit einer schwarzen Substanz imprägniert ist, um das schwarz gefärbte mit Kohlenstofffasern gemischte Papier zu bilden.

12. Fern-Infrarot-Strahler nach Anspruch 10, worin eine schwarze Substanz auf das mit Kohlenstofffasern gemischte Papier (100a) aufgetragen ist, um das schwarz gefärbte mit Kohlenstofffasern gemischte Papier zu bilden.

13. Fern-Infrarot-Strahler nach einem der Ansprüche 10 bis 12, worin die Schicht aus der organischen Verbindung (101, 102) eine oder eine Vielzahl von Harzschichten (101A,B,C,d,e,f) umfasst, um das mit Kohlenstofffasern gemischte Papier bahnförmig auszubilden.

14. Fern-Infrarot-Strahler, umfassend:

    (a) ein wärmeerzeugendes Element, das mit Kohlenstofffasern gemischtes Papier (100) umfasst, worin Elektroden (103) auf dem mit Kohlenstofffasern gemischten Papier vorgesehen sind; und

    (b) eine Schicht aus der organischen Verbindung (101a, 102a), die auf das mit Kohlenstofffasern gemischte Papier auflaminiert ist,

    **dadurch gekennzeichnet, dass** die Schicht aus der organischen Verbindung (101a, 102a) schwarz gefärbt ist.

15. Fern-Infrarot-Strahler nach Anspruch 14, worin eine schwarze Substanz in die Schicht aus der organischen Verbindung (101a, 102) eingemischt und darin dispergiert ist, um die schwarz gefärbte Schicht aus der organischen Verbindung zu bilden.

**16.** Fern-Infrarot-Strahler nach Anspruch 14, worin eine schwarze Substanz auf die Schicht aus der organischen Verbindung (101a, 102a) aufgebracht ist, um die schwarz gefärbte Schicht aus der organischen Verbindung zu bilden.

**17.** Fern-Infrarot-Strahler nach einem der Ansprüche 14 bis 16, worin die Schicht aus der organischen Verbindung (101a, 102a) eine schwarz gefärbte Harzschicht umfasst, wodurch das mit Kohlenstofffasern gemischte Papier bahnförmig ausgebildet ist.

**Revendications**

**1.** Procédé pour produire des radiations dans l'infrarouge lointain, comprenant la génération de rayons dans l'infrarouge lointain sur une plage de longueurs d'onde pleine dans l'infrarouge lointain avec un élément générateur de chaleur comportant du papier mélangé avec des fibres de carbone (100a) et à amener un composé organique sélectionné à absorber ladite radiation dans l'infrarouge lointain et à faire rayonner par résonance ladite radiation dans l'infrarouge lointain dans une plage de longueurs d'onde sélectionnée qui dépend d'un état moléculaire dans ledit composé organique sélectionné, **caractérisé en ce que** le papier mélangé avec des fibres de carbone (100a) est coloré en noir en imprégnant une substance noire dans le papier mélangé avec des fibres de carbone ou en appliquant ou en revêtant une substance noire sur ledit papier mélange avec des fibres de carbone.

**2.** Procédé selon la revendication 1, où ledit composé organique sélectionné est présent comme une couche de composé organique (101, 102), et ladite couche comporte une ou plusieurs couches de résine (101A, B, C, d, e, f) laminées sur ledit papier (100a) mélangé avec des fibres de carbone.

**3.** Procédé selon la revendication 2, où une ou plusieurs couches de résine précitées comportent un laminé de plusieurs types de couches de résine (101A, B, C).

**4.** Procédé selon la revendication 1, où plusieurs composés organiques différents sont amenés à absorber ladite radiation dans l'infrarouge lointain et à faire rayonner par résonance la radiation dans l'infrarouge lointain dans des plages de longueurs d'onde sélectionnées respectives.

**5.** Procédé selon la revendication 4, où plusieurs composés organiques différents sont présents comme plusieurs couches, lesdites couches comprenant plusieurs couches de résine (101A, B, C, d, e, f) laminées sur ledit papier mélangé avec des fibres de carbone.

**6.** Procédé selon la revendication 5, où plusieurs couches de résine comportent plusieurs types de couches de résine (101d, e, f) agencées sur le même plan.

**7.** Procédé pour produire une radiation dans l'infrarouge lointain, comprenant la génération de rayons dans l'infrarouge lointain sur une plage de longueurs d'onde complète dans l'infrarouge lointain avec un élément générateur de chaleur comportant des fibres de carbone (100, 200) et à amener une couche de composé organique (101a, 102a) à absorber lesdits rayons rayonnés dans l'infrarouge lointain et à faire rayonner par résonance les rayons dans l'infrarouge lointain dans une plage de longueurs d'onde sélectionnée qui dépend d'un état moléculaire dans ladite couche de composé organique (101a, 102a) d'une surface de ladite couche de composé organique, **caractérisé en ce que** la couche de composé organique est colorée en noir en mélangeant et en dispersant une substance noire dans la couche de composé organique ou en appliquant à ou en réalisant un revêtement d'une substance noire sur la couche de composé organique.

**8.** Procédé selon la revendication 7, où ledit élément générateur de chaleur comporte du papier (100) mélangé avec des fibres de carbone.

**9.** Procédé selon la revendication 8, où la couche de composé organique colorée en noir (101a, 102a) comporte une ou plusieurs couches de résine laminées sur ledit papier mélangé avec des fibres de carbone.

**10.** Emetteur de radiations dans l'infrarouge lointain (1), comprenant:

(a) un élément générateur de chaleur comportant du papier (100a) mélangé avec des fibres de carbone, où des électrodes (103, 107) sont prévues sur le papier mélangé avec des fibres de carbone; et
(b) une couche de composé organique (101, 102) laminée sur ledit papier mélangé avec des fibres de carbone,

**caractérisé en ce que** le papier (100a) mélangé avec des fibres de carbone est coloré en noir.

**11.** Emetteur de radiations dans l'infrarouge lointain selon la revendication 10, où une substance noire est imprégnée dans ledit papier (100a) mélangé avec des fibres de carbone pour former un papier coloré en noir mélangé avec des fibres de carbone.

**12.** Emetteur de radiations dans l'infrarouge lointain selon la revendication 10, où une substance noire est appliquée sur ledit papier (100a) mélangé avec des fibres de carbone pour former le papier coloré en noir mélangé avec des fibres de carbone.

**13.** Emetteur de radiations dans l'infrarouge lointain selon l'une des revendications 10 à 12, où ladite couche de composé organique (101, 102) comporte une ou plusieurs couches de résine (101A, B, C, d, e, f) pour former ledit papier mélangé avec des fibres de carbone en une forme de feuille.

**14.** Emetteur de radiations dans l'infrarouge lointain (1) comprenant:

(a) un élément générateur de chaleur comportant du papier (100) mélangé avec des fibres de carbone, où des électrodes (103) sont prévues sur le papier mélangé avec des fibres de carbone; et
(b) une couche de composé organique (101a, 102a) laminée sur ledit papier mélangé avec des fibres de carbone,

**caractérisé en ce que** la couche de composé organique (101a, 102a) est colorée en noir.

**15.** Emetteur de radiations dans l'infrarouge lointain selon la revendication 14, où une substance noire est mélangée avec et dispersée dans ladite couche de composé organique (101a, 102a) pour former la couche de composé organique colorée en noir.

**16.** Emetteur de radiations dans l'infrarouge lointain selon la revendication 14, où une substance noire est appliquée sur ladite couche de composé organique (101a, 102a) pour former la couche de composé organique colorée en noir.

**17.** Emetteur de radiations dans l'infrarouge lointain selon l'une des revendications 14 à 16, où ladite couche de composé organique (101a, 102a) comporte une couche de résine colorée en noir configurant ledit papier mélangé avec des fibres de carbone en une forme de feuille.

FIG. 1

〔SPECTRAL RADIATION ENERGY CURVE OF BLACK BODY〕

SPECTRAL RADIATION (ENERGY) DIVERGENCE $(W/cm^2 \cdot \mu m)$

WAVELENGTH $(\mu m)$

EP 0 808 640 B1

F I G. 2

(EMISSIVITY, TEMPERATURE
CHARACTERISTICS OF MATERIALS)

# FIG. 3

[INFRARED ABSORPTION SPECTRUM
OF MATERIALS]

⟶ WAVELENGTH (μm)

TRANSMITTANCE

↕

ABSORPTANCE

3600 2300 2000 1800 1600 1400 1200 1000 800 600

⟶ WAVENUMBER (cm⁻¹)

METHYL METHACRYLATE RESIN

EPOXY RESIN

PHENOL RESIN

MELAMINE RESIN

NITROCELLULOSE

VINYL CHLORIDE RESIN

VINYL ACETATE RESIN

17

FIG. 4
[REFLECTION CHARACTERISTICS OF METAL LUSTER SURFACES]

FIG. 5A

FIG. 5B

101.ORGANIC COMPOUND LAYER

100a.CARBON FIBER MIXED PAPER

102.ORGANIC COMPOUND LAYER

FIG. 6

101A
101B } 101
101C

100a    102

F I G. 7

1

101.ORGANIC COMPOUND LAYER

100a.CARBON FIBER MIXED PAPER

107  106.REFLECTION PLATE  107

F I G. 8

1

101.ORGANIC COMPOUND LAYER

107  100a.CARBON FIBER MIXED PAPER  107

F I G. 9

F I G. 1 0

FIG. 11A

103    100    101a    103

FIG. 11B

105    101a.ORGANIC COMPOUND LAYER

103    100.CARBON FIBER
MIXED PAPER

104    102a.ORGANIC COMPOUND LAYE

FIG. 12

101a.ORGANIC COMPOUND LAYER

107    200.CARBON FIBER    107

22

FIG. 13